(19) Europäisches Patentamt / European Patent Office / Office européen des brevets

(11) **EP 3 803 330 B1**

(12) **EUROPÄISCHE PATENTSCHRIFT**

(45) Veröffentlichungstag und Bekanntmachung des Hinweises auf die Patenterteilung:
**06.04.2022 Patentblatt 2022/14**

(21) Anmeldenummer: **19729272.5**

(22) Anmeldetag: **06.06.2019**

(51) Internationale Patentklassifikation (IPC):
**G01N 11/00** (2006.01)     **G01N 11/04** (2006.01)
**G01F 1/66** (2022.01)     **G01N 33/49** (2006.01)
**A61B 5/00** (2006.01)

(52) Gemeinsame Patentklassifikation (CPC):
**G01N 11/04; A61B 5/0031; A61B 5/14503;**
**G01N 33/49;** G01F 1/663; G01N 2011/0073

(86) Internationale Anmeldenummer:
**PCT/EP2019/064810**

(87) Internationale Veröffentlichungsnummer:
**WO 2019/234169 (12.12.2019 Gazette 2019/50)**

(54) **ANALYSEVORRICHTUNG UND VERFAHREN ZUM ANALYSIEREN EINER VISKOSITÄT EINES FLUIDS**

ANALYSIS APPARATUS AND METHOD FOR ANALYZING A VISCOSITY OF A FLUID

DISPOSITIF D'ANALYSE ET PROCÉDÉ POUR ANALYSER LA VISCOSITÉ D'UN FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018 DE 102018208945**

(43) Veröffentlichungstag der Anmeldung:
**14.04.2021 Patentblatt 2021/15**

(73) Patentinhaber: **Kardion GmbH**
**70376 Stuttgart (DE)**

(72) Erfinder:
- **STOTZ, Ingo**
  **71254 Ditzingen (DE)**
- **SCHLEBUSCH, Thomas, Alexander**
  **71272 Renningen (DE)**
- **SCHMID, Tobias**
  **70197 Stuttgart (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
EP-A1- 2 175 770      DE-A1-102006 001 180
US-A1- 2004 065 143     US-A1- 2005 126 268
US-A1- 2012 084 024

- **Volkan Osel ET AL: "Online Viscosity Measurement of Complex Solutions Using Ultrasound Doppler Velocimetry", Turk J Chem c UB &dot; ITAK, 1. Januar 2006 (2006-01-01), Seiten 297-305, XP055607565, Gefunden im Internet: URL:http://signal-processing.ch/download/on_line_visco.pdf**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 803 330 B1

## Beschreibung

Die Erfindung betrifft ein Herzunterstützungssystem mit einer Aufnahmeschnittstelle, mit Einströmöffnungen und mit einer Auslassschnittstelle mit Austrittsöffnungen, mit einer Strömungsmaschine, die einen Impeller aufweist, der mit einem Elektromotor gekoppelt ist, mit einem Anschlusskabel und mit einer an das Anschlusskabel angeschlossenen Kanüle, die entlang einer Achse erstreckt und mittig in Aortenklappen anordenbar ist, durch die ein Blutstrom mittels der Strömungsmaschine von der Aufnahmeschnittstelle zu der Auslassschnittstelle gefördert werden kann. Darüber hinaus betrifft die Erfindung ein Verfahren zum Analysieren der Viskosität des Bluts eines durch eine Kanüle geförderten Blutstroms, die entlang einer Achse erstreckt ist.

PT (Prothrombinzeit) und INR (International Normalized Ratio) sind das Standardmaß für die Blutgerinnung. Üblicherweise wird das INR in Blutproben durch Zugabe von Thromboplastin und anschließender Messung der Zeit bis zur Gerinnung bestimmt. Die Bestimmung kann im Labor erfolgen, es sind mittlerweile auch Teststreifengeräte zur Selbstmessung durch den Patienten verfügbar, vergleichbar mit dem Ablauf einer Blutzuckermessung. Für Patienten mit Herzunterstützungssystemen ist das sogenannte Gerinnungsmanagement zur Minimierung von Pumpenthrombosen essenziell. Möglicherweise ist für das Gerinnungsmanagement die Überwachung der Blutviskosität als INR-Ersatzparameter ausreichend.

In Volkan Osel et al.: Oline Viscosity Measurement of Complex Solutions Using Ultrasound Doppler Velocimetry", Turk. J. Chem. 30, 297 (2006) beschreibt das Bestimmen der Viskosität einer Flüssigkeit mittels Ultraschall-Doppler-Geschwindigkeitsmessung. Angaben zu in-vivo-Messungen gibt es hier nicht.

Aus der US 2004/065143 A1 ist das Messen der Blutviskosität mit einem Ultraschallelement unter Verwendung eines Dippler-Parameters bekannt.

Die DE 10 2006 001 180 A1 offenbart ein Rheometer und Auswerteverfahren zur Bestimmung von Fließkurve und Viskositätsfunktion von optisch transparenten Newtonschen und Nicht-Newtonschen Flüssigkeiten.

Die US 2012/0084024 A1 beschreibt ein Viskosimeter mit einer Ultraschallmesseinrichtung für das Bestimmen der Durchflussgeschwindigkeit von Fluid durch einen Fluidkanal.

Aus der US 2005/0126268 A1 ist eine Ultraschall-Messvorrichtung für das Bestimmen eines Geschwindigkeitsprofils einer Flüssigkeit bekannt, die durch einen Fluidkanal strömt.

Die EP 2 175 770 B1 beschreibt einen expliziten Blut-Viskositätssensor auf Basis von Oberflächenwellen, kurz SAW, zur Bestimmung der Viskosität. Angaben zum Messen von Doppler-Parametern gibt es hier nicht.

Die US 7,591,777 B2 beschreibt eine Viskositätsbestimmung in Herzunterstützungssystemen durch die mechanische Rückwirkung der Blutviskosität auf den Antrieb des Herzunterstützungssystems.

Aufgabe der Erfindung ist es, ein verbessertes Verfahren zum Analysieren einer Viskosität eines Fluids und eine verbesserte Analysevorrichtung hierzu anzugeben, Insbesondere ist es eine Aufgabe der Erfindung, ein Verfahren und eine Vorrichtung anzugeben, die das Analysieren der Viskosität eines Fluids fortlaufend und auf einer kurzen Zeitskala ermöglicht.

Diese Aufgabe wird durch die in Anspruch 1 angegebene Bestimmvorrichtung und das in Anspruch 13 angegebene Verfahren gelöst. Vorteilhafte Ausführungsformen der Erfindung sind in den abhängigen Ansprüchen angegeben.

Nachfolgend werden eine Analysevorrichtung zum Analysieren einer Viskosität eines Fluids und ein erfindungsgemäßes Verfahren zum Analysieren einer Viskosität eines Fluids sowie schließlich ein entsprechendes Computerprogramm vorgestellt. Durch die in den abhängigen Ansprüchen aufgeführten Maßnahmen sind vorteilhafte Weiterbildungen und Verbesserungen der in den unabhängigen Ansprüchen angegebenen Gegenstände möglich.

Vor diesem Hintergrund werden mit dem hier vorgestellten Ansatz eine Analysevorrichtung zum Analysieren einer Viskosität eines Fluids und ein Verfahren zum Analysieren einer Viskosität eines Fluids sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Die mit dem vorgestellten Ansatz erreichbaren Vorteile bestehen darin, dass eine hier vorgestellte Analysevorrichtung dazu ausgebildet ist, um die Viskosität eines Fluids schnell und einfach unter Verwendung eines aktuellen Doppler-Parameters des Fluids zu erkennen und bereitzustellen oder zu senden. Unter einem Doppler-Parameter kann hierbei ein Parameter verstanden werden, der eine Information über eine Veränderung einer Frequenz eines in das Fluid ausgesandten Signals zu einer Freqzuenz eines aus dem Fluid empfangenen Signals repräsentiert. Beispielsweise entspricht der Doppler-Parameter einer Doppler-Verschiebung. Unter einem Doppler-Spektrum kann vorliegend ein Spektrum verstanden werden, welches Frequenzen enthält, die von einem in das Fluid ausgesandten Signal resultieren, sowie Frequenzen enthält, die von einem aus dem Fluid empfangenen Signal resultieren. Auf diese Weise kann beispielsweise eine Auswertung der Doppler-Verschiebung von unterschiedlichen Frequenzanteilen von in das Fluid ausgesandten Signalen in Bezug auf die Frequenzanteile, die von aus dem Fluid empfangenen Signalen resultieren, ermöglicht werden.

Es wird eine Analysevorrichtung zum Analysieren einer Viskosität eines Fluids vorgestellt. Die Analysevorrichtung weist eine Erkenneinrichtung und eine Bereitstellungseinrichtung auf. Die Erkenneinrichtung ist dazu ausgebildet, unter Verwendung zumindest eines Doppler-Parameters eines Doppler-Spektrums des Fluids die Viskosität des Fluids zu erkennen. Die Bereitstellungseinrichtung ist dazu ausgebildet, um ein Viskositätssignal bereitzustellen oder zu senden, das die von der Erkenneinrichtung erkannte Viskosität repräsentiert. Als das Doppler-Spektrum ist ein Produkt aus einem Strömungsprofil des Fluids und einer Richtcharakteristik eines Ultraschallelements, welches eine Schallwelle in dem Fluid erzeugt oder erzeugen kann, zu verstehen. Das Strömungsprofil kann abhängig sein von einer Strömungsgeschwindigkeit des Fluids und zusätzlich oder alternativ von einer Ausformung einer Aufnahmeeinrichtung, durch welche das Fluid strömt.

Die Erkenneinrichtung kann dazu ausgebildet sein, um den Doppler-Parameter von einem solchen Ultraschallelement einzulesen, welches ein Ultraschallwandler sein kann. Das Ultraschallelement kann dazu ausgebildet sein, um die Schallwelle in dem Fluid zu erzeugen und den Doppler-Parameter einer zurückkommenden reflektierten Schallwelle in dem Fluid zu sensieren. Die erzeugte Schallwelle kann eine definierte oder feste Richtcharakteristik aufweisen. Die Erkenneinrichtung und/oder die Bereitstellungseinrichtung kann hierbei Teil des Ultraschallelements sein oder mit diesem koppelbar ausgebildet sein. Beispielsweise kann die Erkenneinrichtung dazu ausgebildet sein, um den von dem Ultraschallelement sensierten Doppler-Parameter von dem Ultraschallelement einzulesen.

Die Erkenneinrichtung kann dazu ausgebildet sein, um die Viskosität unter Verwendung eines Funktionszusammenhangs zwischen dem Doppler-Parameter zu der Viskosität und/oder unter Verwendung einer Nachschlagetabelle zu erkennen, insbesondere wobei in der Nachschlagetabelle ein Zusammenhang zwischen dem Doppler-Parameter zu der Viskosität abgelegt sein kann. Die Nachschlagetabelle kann eine Kalibriertabelle sein, in der Messdaten für alle relevanten Viskositäten des Fluids bei allen relevanten Doppler-Parametern und zusätzlich oder alternativ weitere relevante Parameter wie Strömungsgeschwindigkeiten des Fluids abgelegt sein können. So kann unter Verwendung des aktuellen Doppler-Parameters schnell und einfach aus der Nachschlagetabelle eine diesem zugeordnete Viskosität ausgelesen werden. Oder es kann unter Verwendung des aktuellen Doppler-Parameters schnell und einfach durch Lösen des Funktionszusammenhangs die Viskosität erkannt werden. Die Nachschlagetabelle und/oder der Funktionszusammenhang kann in der Erkenneinrichtung gespeichert sein oder von der Erkenneinrichtung zur Verwendung einlesbar sein.

Es ist weiterhin von Vorteil, wenn die Erkenneinrichtung gemäß einer Ausführungsform dazu ausgebildet ist, um die Viskosität unter Verwendung einer Interpolation einer in der Nachschlagetabelle abgelegten ersten Viskosität und einer in der Nachschlagetabelle abgelegten (angrenzenden) zweiten Viskosität zu erkennen. So kann eine Berechnungsgenauigkeit gesteigert werden.

Die Analysevorrichtung kann zudem eine Kanüle mit einer Aufnahmeschnittstelle zur Aufnahme des Fluids und einer der Aufnahmeschnittstelle gegenüberliegenden Auslassschnittstelle zum Auslassen des Fluids aufweisen, insbesondere wobei der Doppler-Parameter einen Doppler-Parameter in der Kanüle repräsentieren kann. Eine derartige Kanüle kann zur Verwendung an oder in einem Herzunterstützungssystem ausgeformt sein. Beispielsweise kann die Kanüle dazu ausgeformt oder ausgebildet sein, um als das Fluid Blut aufzunehmen. So kann vorteilhafterweise unter Verwendung der Analysevorrichtung die aktuelle Viskosität des Bluts in der Kanüle erkannt werden. Die Erkenneinrichtung kann zudem dazu ausgebildet sein, um die Viskosität unter Verwendung zumindest eines Kanülenparameters der Kanüle zu erkennen. Der Kanülenparameter kann eine Kanülenbreite oder ein Kanülenradius sein.

Gemäß einer weiteren vorteilhaften Ausführungsform weist die Analysevorrichtung eine Strömungseinrichtung zum Fördern des Fluids von der Aufnahmeschnittstelle zu der Auslassschnittstelle der Kanüle auf, insbesondere wobei die Strömungseinrichtung an oder im Bereich der Auslassschnittstelle angeordnet oder anordenbar sein kann. Die Strömungseinrichtung kann eine Antriebseinrichtung in Form eines Elektromotors und einen gekoppelten Impeller aufweisen. Im Betrieb der Strömungseinrichtung kann so ein Volumenstrom des Fluids durch die Kanüle bewirkt werden, wobei der Volumenstrom das Strömungsprofil messbar macht, welches abhängig ist von der Viskosität des Fluids, einer Strömungsgeschwindigkeit des Fluids und einer Ausformung der Kanüle, beispielsweise der Kanülenbreite oder des Kanülenradius. Eine derartige Analysevorrichtung mit Strömungseinrichtung kann als ein Herzunterstützungssystem ausgeformt oder einsetzbar sein. Dieses Herzunterstützungssystem kann vorteilhafterweise eine aktuelle Blutviskosität erkennen und beispielsweise für ein Diagnoseverfahren bereitstellen oder senden.

Die Erkenneinrichtung kann zudem dazu ausgebildet sein, um die Viskosität unter Verwendung zumindest eines Strömungsparameters des Strömungsprofils, insbesondere einer Strömungsgeschwindigkeit, des Fluids durch die Kanüle zu erkennen. Die Strömungsgeschwindigkeit kann unter Verwendung eines Ultraschallelements, das dazu ausgebildet ist, um die Dopplerverschiebung des an Partikeln des Fluids reflektierten Ultraschallsignals zu sensieren, messbar sein.

Es ist weiterhin von Vorteil, wenn die Analysevorrichtung gemäß einem Ausführungsbeispiel ein Ultraschallelement aufweist, das dazu ausgebildet ist, um in dem Fluid eine Schallwelle zu erzeugen, um den Doppler-Parameter zu erkennen, insbesondere wobei das Ultraschallelement im Bereich der Aufnahmeschnittstelle der Kanüle angeordnet sein kann. Das Ultraschallelement kann dazu ausgebildet sein, um die Schallwelle mit einer definierten oder festen Richtcharakteristik zu erzeugen. Hierbei kann die Richtcharakteristik in Richtung der zu erwartenden Fluidströmung des Fluids durch die Kanüle ausgerichtet sein.

Die Erkenneinrichtung kann dazu ausgebildet sein, um die Viskosität unter Verwendung des Doppler-Parameters zu erkennen, der eine Doppler-Frequenz und/oder eine Breite des Doppler-Spektrums repräsentiert.

Es wird zudem ein Verfahren zum Analysieren einer Viskosität eines Fluids vorgestellt. Das Verfahren umfasst einen Schritt des Erkennens und einen Schritt des Bereitstellens. Im Schritt des Erkennens wird die Viskosität des Fluids unter Verwendung zumindest eines Doppler-Parameters eines Doppler-Spektrums des Fluids erkannt. Im Schritt des Bereitstellens wird ein Viskositätssignal bereitgestellt oder gesendet, das die im Schritt des Erkennens erkannte Viskosität repräsentiert.

Dieses Verfahren kann unter Verwendung der vorangehend vorgestellten Analysevorrichtung durchführbar sein. Das Verfahren kann beispielsweise in Software oder Hardware oder in einer Mischform aus Software und Hardware beispielsweise in einem Steuergerät implementiert sein.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:

Fig. 1 eine schematische Darstellung einer Analysevorrichtung zum Analysieren einer Viskosität eines Fluids gemäß einem Ausführungsbeispiel;

Fig. 2 eine schematische seitliche Querschnittdarstellung einer Analysevorrichtung gemäß einem Ausführungsbeispiel;

Fig. 3 eine schematische Darstellung eines Herzunterstützungssystems mit einer Analysevorrichtung gemäß einem Ausführungsbeispiel;

Fig. 4 eine schematische Darstellung eines Strömungsprofils eines Fluids gemäß einem Ausführungsbeispiel;

Fig. 5 eine schematische Darstellung eines Doppler-Spektrums;

Fig. 6 eine schematische Darstellung eines Doppler-Spektrums;

Fig. 7 eine schematische Darstellung eines Doppler-Spektrums gemäß einem Ausführungsbeispiel; und

Fig. 8 ein Ablaufdiagramm eines Verfahrens zum Analysieren einer Viskosität eines Fluids gemäß einem Ausführungsbeispiel.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

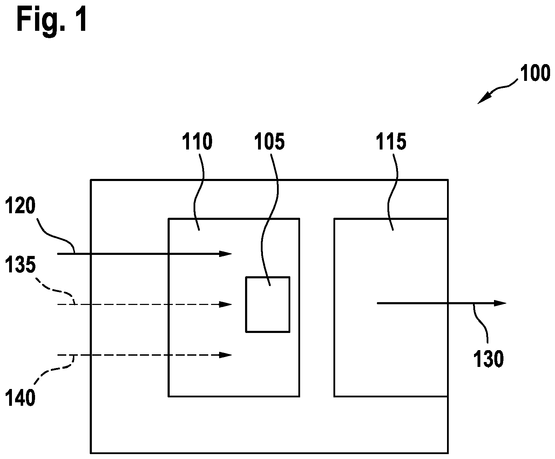

Fig. 1 zeigt eine schematische Darstellung einer Analysevorrichtung 100 zum Analysieren einer Viskosität 105 eines Fluids gemäß einem Ausführungsbeispiel.

Die Analysevorrichtung 100 weist eine Erkenneinrichtung 110 und eine Bereitstellungseinrichtung 115 auf. Die Erkenneinrichtung 110 ist dazu ausgebildet, unter Verwendung zumindest eines Doppler-Parameters 120 eines Doppler-Spektrums des Fluids die Viskosität 105 des Fluids zu erkennen. Die Bereitstellungseinrichtung 115 ist dazu ausgebildet, um ein Viskositätssignal 130 bereitzustellen oder zu senden, das die von der Erkenneinrichtung 110 erkannte Viskosität 105 repräsentiert.

Gemäß diesem Ausführungsbeispiel ist die Erkenneinrichtung 110 dazu ausgebildet, die Viskosität 105 unter Verwendung eines Strömungsparameters 135 des Fluids durch eine Kanüle, in der das Fluid aufgenommen ist, und/oder eines Kanülenparameters 140 der Kanüle zu erkennen. Die Erkenneinrichtung 110 ist gemäß diesem Ausführungsbeispiel dazu ausgebildet, den Doppler-Parameter 120 und/oder den Strömungsparameter 135 und/oder den Kanülenparameter 140 in Form von je einem Sensorsignal einzulesen.

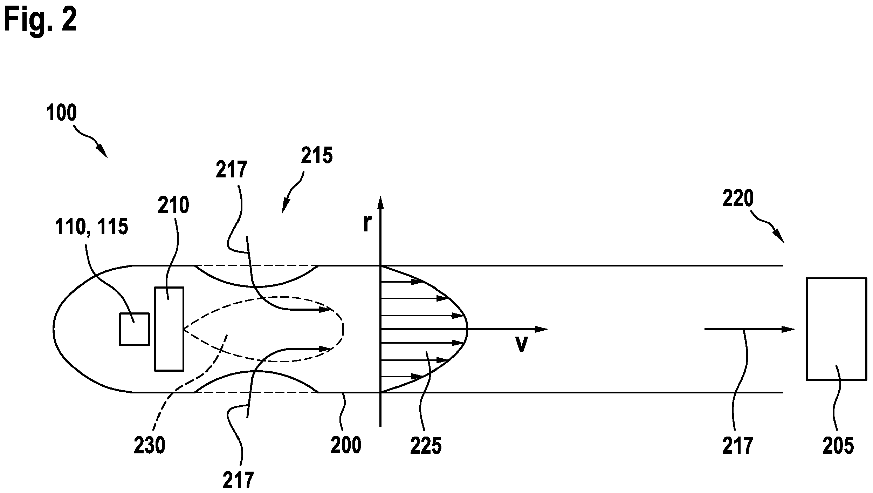

Fig. 2 zeigt eine schematische seitliche Querschnittdarstellung einer Analysevorrichtung 100 gemäß einem Ausführungsbeispiel. Dabei kann es sich um die in Figur 1 beschriebene Analysevorrichtung 100 handeln, mit dem Unterschied, dass die Analysevorrichtung 100 gemäß diesem Ausführungsbeispiel zusätzlich eine Kanüle 200, eine Strömungseinrichtung 205 und ein Ultraschallelement 210 aufweist. Alternativ oder zusätzlich kann die Analysevorrichtung 100 beispielsweise zweiteilig ausgeführt werden, sodass die Kanüle 200, die Strömungseinrichtung 205 und das Ultraschallelement 210 räumlich durch ein Kabel von der Erkenneinrichtung 110 und der Bereitstellungseinrichtung 115 separiert betrieben werden können.

Die Kanüle 200 weist eine Aufnahmeschnittstelle 215, die dazu ausgeformt ist, das Fluid 217 aufzunehmen, und eine der Aufnahmeschnittstelle 215 gegenüberliegende Auslassschnittstelle 220 auf, die dazu ausgebildet ist, um das Fluid 217 auszulassen. Der Doppler-Parameter repräsentiert gemäß diesem Ausführungsbeispiel einen Doppler-Parameter in der Kanüle 200.

Die Strömungseinrichtung 205 ist dazu ausgebildet, das Fluid 217 von der Aufnahmeschnittstelle 215 zu der Auslassschnittstelle 220 der Kanüle 200 zu fördern. Hierzu ist die Strömungseinrichtung 205 gemäß diesem Ausführungsbeispiel an oder im Bereich der Auslassschnittstelle 220 angeordnet oder anordenbar. Gemäß diesem Ausführungsbeispiel weist die Strömungseinrichtung 205 eine Antriebseinrichtung in Form eines Elektromotors und/oder einen gekoppelten Impeller auf, der in der Kanüle 200 aufgenommen ist.

Die Erkenneinrichtung 110 ist gemäß diesem Ausführungsbeispiel dazu ausgebildet, die Viskosität unter Verwendung des Strömungsparameters zu erkennen, der eine Strömungsgeschwindigkeit v eines Strömungsprofils 225 des Fluids durch die Kanüle 200 repräsentiert. Die Erkenneinrichtung 110 ist zudem gemäß diesem Ausführungsbeispiel dazu ausgebildet, die Viskosität unter Verwendung des Kanülenparameters der Kanüle 200 zu erkennen, der eine Kanülenbreite r der Kanüle 200 repräsentiert.

Das Ultraschallelement 210 ist dazu ausgebildet, in dem Fluid 217 eine Schallwelle zu erzeugen, um in deren Reflexion an Partikeln im Fluid den Doppler-Parameter zu erkennen. Das Ultraschallelement 210 ist gemäß diesem Ausführungsbeispiel im Bereich der Aufnahmeschnittstelle 215 der Kanüle 200 angeordnet. Gezeigt ist außerdem eine Richtcharakteristik 230 des Ultraschallelements 210, wobei die Richtcharakteristik 230 gemäß diesem Ausführungsbeispiel fest und/oder definiert ist.

Die Erkenneinrichtung 110 ist gemäß diesem Ausführungsbeispiel dazu ausgebildet, die Viskosität unter Verwendung des Doppler-Parameters zu erkennen, der eine Doppler-Frequenz und/oder eine Breite des Doppler-Spektrums repräsentiert. Gemäß diesem Ausführungsbeispiel ist die Erkenneinrichtung 110 hierbei dazu ausgebildet, die Viskosität unter Verwendung eines Funktionszusammenhangs zwischen dem Doppler-Parameter zu der Viskosität und/oder unter Verwendung einer Nachschlagetabelle zu erkennen, wobei in der Nachschlagetabelle ein Zusammenhang zwischen dem Doppler-Parameter zu der Viskosität abgelegt ist. Die Erkenneinrichtung 110 ist gemäß diesem Ausführungsbeispiel außerdem dazu ausgebildet, die Viskosität unter Verwendung einer Interpolation einer in der Nachschlagetabelle abgelegten ersten Viskosität und einer in der Nachschlagetabelle abgelegten angrenzenden zweiten Viskosität zu erkennen.

Im Folgenden werden Details der Analysevorrichtung 100 noch einmal mit anderen Worten genauer beschrieben:
Die hier vorgestellte Analysevorrichtung 100 ist gemäß diesem Ausführungsbeispiel als ein Herzunterstützungssystem einsetzbar. Für Patienten mit einem Herzunterstützungssystem, auch VAD-Patienten genannt, VAD steht für "Ventricular Assist Device", ist das Gerinnungsmanagement zur Minimierung von Pumpenthrombosen essenziell. Die Patienten werden dazu beispielsweise mit Arzneimitteln zur Hemmung der plasmatischen Blutgerinnung therapiert und der INR wird so beispielsweise im Bereich 2 bis 2,5 eingestellt.

Durch Analyse des Doppler-Spektrums des gemäß diesem Ausführungsbeispiel integrierten Ultraschallelements 210 in einer Spitze der Kanüle 200 eines VAD-Systems, die auch als Zulaufkanüle bezeichnet werden kann, kann auf das Strömungsprofil 225 und damit die Viskosität des Blutes geschlossen werden.

Die Blutviskosität wird gemäß diesem Ausführungsbeispiel im Betrieb der Analysevorrichtung 100 von der Erkenneinrichtung 110 kontinuierlich oder gemäß einem alternativen Ausführungsbeispiel in festen Zeitintervallen erhoben. Die Bereitstellungseinrichtung 115 ist dazu ausgebildet, um die erkannte Viskosität einem Arzt und/oder Patienten als einen Parameter zur Therapieführung bereitzustellen. Hierzu ist das Viskositätssignal dazu ausgebildet, die Viskosität auf einem Display anzuzeigen und/oder durch Funkübertragung in einen Webdienst zu übermitteln.

Vorteilhafterweise ist bei der hier vorgestellten Analysevorrichtung 100 als Ultraschallelement 210 lediglich ein einfacher sogenannter "single-element"-Ultraschallwandler ausreichend, der gemäß diesem Ausführungsbeispiel als eine Kreisscheibe ausgeformt ist. Ein solches Ultraschallelement 210 ist aufgrund der hier gezeigten speziellen räumlichen Positionierung des Ultraschallelements 210 in Richtung der zu erwartenden Strömung des Fluids 217 möglich. Das Ultraschallelement 210 ist gemäß einem Ausführungsbeispiel zur Quantifizierung der Flussgeschwindigkeit v des Fluids 217 ausgebildet.

Das in der Spitze der Zulaufkanüle integrierte Ultraschallelement 210 misst zum Beispiel über das sogenannte "Pulsed-wave-Doppler"-Verfahren, dieses Verfahren wird auch als "Gepulster Doppler" bezeichnet, das Doppler-Spektrum der Strömung in der Kanüle 200.

Gezeigt ist in Figur 2 anders ausgedrückt eine beispielhafte Ausführung einer VAD-Zulaufkanüle mit Ultraschallelement 210 in Form eines Ultraschallwandler. Gezeigt sind ein Zulaufbereich, die Richtcharakteristik 230 des Ultraschallwandlers und das sich einstellende Strömungsprofil 225 in der Zulaufkanüle.

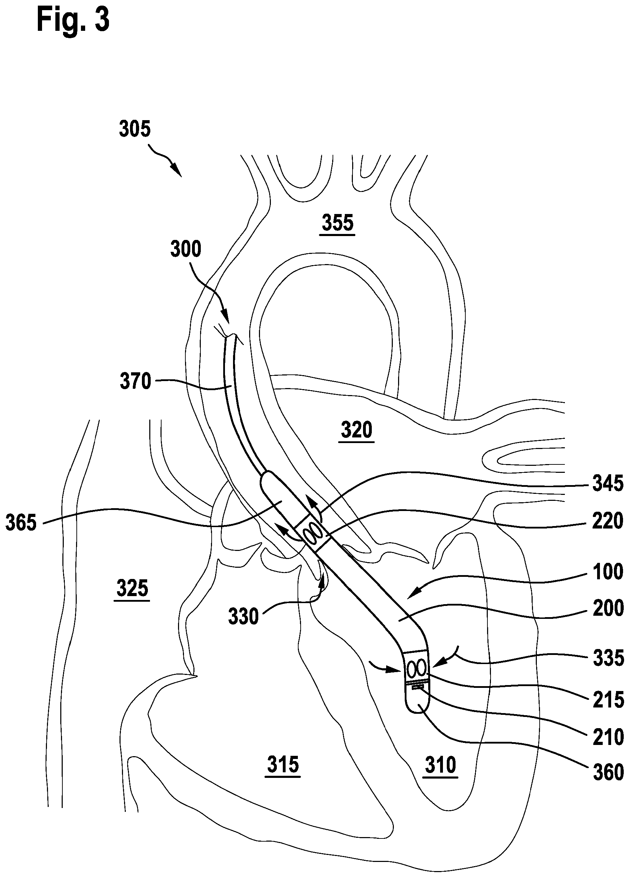

Fig. 3 zeigt eine schematische Darstellung eines Herzunterstützungssystems 300 mit einer Analysevorrichtung 100 gemäß einem Ausführungsbeispiel. Dabei kann es sich um die anhand von Figur 2 beschriebene Analysevorrichtung 100 handeln.

Das hier exemplarisch dargestellte Herzunterstützungssystem 300 kann auch als ein kardiales Unterstützungssystem bezeichnet werden. Gezeigt ist zudem ein Herz 305 mit linkem Ventrikel 310 und rechtem Ventrikel 315 sowie linkem Vorhof 320 und rechtem Vorhof 325. Das Herzunterstützungssystem 300 liegt mittig in Aortenklappen 330, sodass ein Blutstrom 335 durch die Aufnahmeschnittstelle 215 in Form von Einströmöffnungen im Bereich des linken Ventrikels 310 angesaugt und im Bereich nach den Herzklappen 345 durch die Auslassschnittstelle 220 in Form von Austrittsöffnungen in die Aorta 355 abgegeben wird.

Das exemplarische Unterstützungssystem umfasst gemäß diesem Ausführungsbeispiel zudem eine distale Spitze 360 mit Sensoren, gemäß einem Ausführungsbeispiel umfassen die Sensoren zumindest einen Druck- und/oder zumindest einen Temperatursensor, sowie dem Ultraschallelement 210, das entlang der Achse des Unterstützungssystems durch einen Zulaufbereich der Aufnahmeschnittstelle 215 hindurch in die Kanüle 200 hineinstrahlt. Die Kanüle 200 leitet das Blut zur Strömungsmaschine mit Impeller, die sich im Bereich der Auslassschnittstelle 220 befinden. Daran anschließend befinden sich ein Elektromotor 365 und ein Anschlusskabel 370.

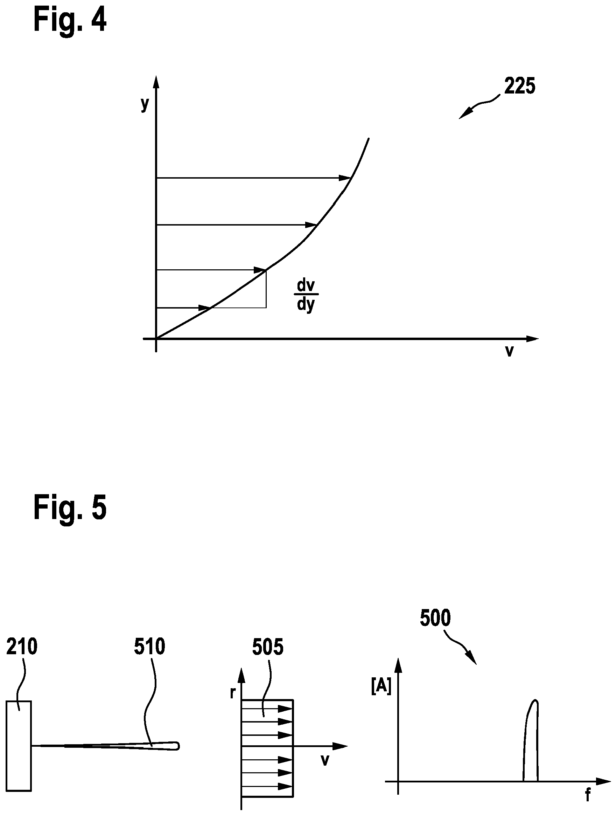

Fig. 4 zeigt eine schematische Darstellung eines Strömungsprofils 225 eines Fluids gemäß einem Ausführungsbeispiel. Dabei kann es sich um das in Figur 2 beschriebene Strömungsprofil 225 handeln, welches von einer der Analysevorrichtungen erkennbar ist, die in einer der vorangegangenen Figuren beschrieben wurde. Gezeigt ist ein beispielhaftes Strömungsprofil 400 in einer Röhre, wobei v eine Geschwindigkeit des Fluids bezeichnet und y einen radialen Abstand von einer Rohrinnenwand der Röhre. Der Geschwindigkeitsgradient $\partial v / \partial y$, und damit das Geschwindigkeitsprofil, ist viskositätsabhängig. Anders ausgedrückt ist das Geschwindigkeitsprofil in einer Kanüle eines Herzunterstützungssystems nach Navier-Stokes von der Viskosität abhängig.

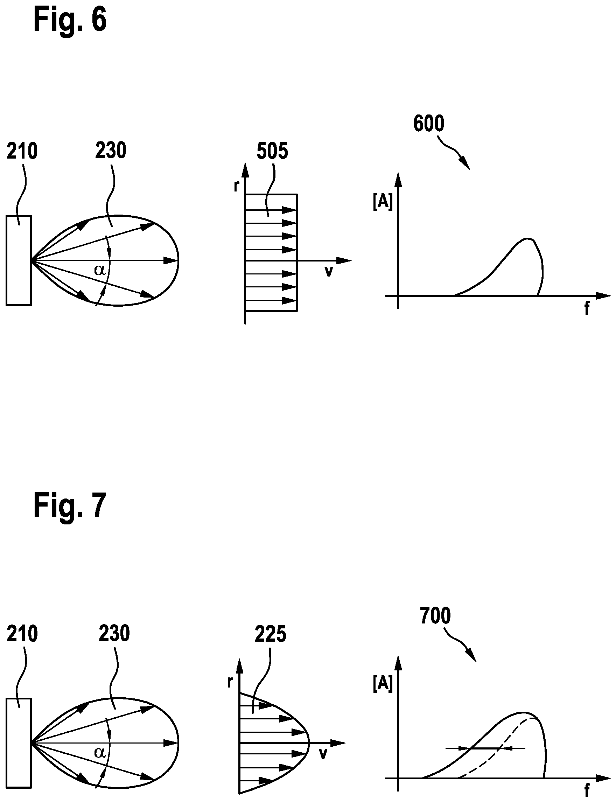

Fig. 5 zeigt eine schematische Darstellung eines Doppler-Spektrums 500. Das Doppler-Spektrum 500 ist das Produkt aus Strömungsprofil 505 eines Fluids und Richtcharakteristik 510 eines Ultraschallelements 210. In den Figuren 5 bis 7 sind unterschiedliche Strömungsprofile und Richtcharakteristika sowie jeweils resultierende Doppler-Spektren gegenübergestellt, wobei Figur 7 ein reales Doppler-Spektrum zeigt, wie es unter Verwendung der in einer der Figuren 1 bis 3 vorgestellten Analysevorrichtungen bewirkt wird und/oder erkennbar ist.

Figur 5 zeigt ein Doppler-Spektrum 500 für ein ideal fokussierendes Ultraschallelement 210, welches eine ideale Richtcharakteristik 510 bewirkt, und eine parallele Strömung, welche das parallele Strömungsprofil 505 ergibt.

Fig. 6 zeigt eine schematische Darstellung eines Doppler-Spektrums 600. Gezeigt ist ein resultierendes Doppler-Spektrum 600 für ein real fokussierendes Ultraschallelement 210, das die zur Verwendung mit der in einer der Figuren 1 bis 3 beschriebenen Analysevorrichtung beschriebene Richtcharakteristik 230 bewirkt, und das in Figur 5 beschriebene parallele Strömungsprofil 505. Gegenüber dem in Figur 5 gezeigten Doppler-Spektrum ist das in Figur 6 resultierende Doppler-Spektrum 600 aufgeweitet.

Fig. 7 zeigt eine schematische Darstellung eines Doppler-Spektrums 700 gemäß einem Ausführungsbeispiel. Dabei kann es sich um das Doppler-Spektrum 700 handeln, wie es unter Verwendung der in einer der Figuren 1 bis 3 vorgestellten Analysevorrichtungen in der Kanüle bewirkt wird und/oder erkennbar ist.

Gezeigt ist ein resultierendes Doppler-Spektrum 700 des Fluids für das real fokussierende Ultraschallelement 210, das eine reale Richtcharakteristik 230 aufweist, und ein reales Strömungsprofil 225, wie es sich in der Kanüle ergibt.

Für höhere Viskositäten kommt es zu einer weiteren Aufweitung des Doppler-Spektrums 700, da bei gegebenem Volumenstrom des Fluids die Strömung in der Mitte schneller strömt und am Rand langsamer und die Bereiche langsamer Strömung mehr Querschnittsfläche im Fokusbereich des Ultraschallelements 210 einnehmen.

Die Doppler-Frequenzverschiebungen aller im Strömungsprofil 225 vorkommenderund im Doppler-Spektrum dargestellter Geschwindigkeiten $v_i$ betragen:

$$\Delta f_i = f_0 \frac{2v_i}{c} cos(\alpha_i)$$

Der Peak im Doppler-Spektrum 700 repräsentiert die dominante Geschwindigkeit, das heißt analog zu einem Histogramm eine häufigste auftretende Geschwindigkeit. Allerdings ist dieser Wert noch mit der Richtcharakteristik 230 des Ultraschallelements 210 gewichtet, der nicht in alle Richtungen gleich sensitiv arbeitet.

Die höchste auftretende Doppler-Frequenz repräsentiert die höchste auftretende Geschwindigkeit, da diese aufgrund des speziellen mechanischen Aufbaus in der Hauptstrahlrichtung des Ultraschallelements 210 zu erwarten ist, da:

$$\alpha_{0°} = 0 \rightarrow cos(\alpha_{0°}) = 1.$$

Für ein gegebenes Ultraschallelement 210 mit fester Richtcharakteristik 230 korreliert eine Breite des Doppler-Spektrums 700 mit einer Geschwindigkeitsverteilung im Beobachtungsraum. Als Berechnungsmaß der Erkenneinrichtung für die Viskosität dienen Kennzahlen des Doppler-Spektrums 700, gemäß einem Ausführungsbeispiel auf Basis der Parameter Doppler-Frequenz bei halber Maximalamplitude des Doppler-Spektrums 700 und/oder Breite des Doppler-Spektrums 700 bei gemäß einem Ausführungsbeispiel beispielhaften 90% des Spitzenwerts und/oder Frequenz der Maximalamplitude des Doppler-Spektrums 700 und maximaler Dopplerfrequenz im Doppler-Spektrum 700.

Die Berechnung oder das Erkennen der Viskosität erfolgt gemäß einem Ausführungsbeispiel durch die Erkenneinrichtung recheneffizient unter Verwendung einer Nachschlagetabelle oder Kalibriertabelle, einer sogenannten "Lookup-Tabelle", kurz LUT, in der Messdaten für alle relevanten Viskositäten bei allen relevanten Strömungsgeschwindigkeiten abgelegt sind. Auf Basis der dominanten Doppler-Frequenz wird gemäß einem Ausführungsbeispiel eine Spalte für die dominante Flussgeschwindigkeit selektiert und dort gemäß einem Ausführungsbeispiel entsprechend der Breite des Doppler-Spektrums 700 die Viskosität abgelesen. Durch Interpolation zwischen angrenzenden Tabelleneinträgen wird gemäß einem Ausführungsbeispiel die Berechnungsgenauigkeit weiter gesteigert.

Eine Verwendung des Strömungsprofils 225 der hier vorgestellten Analysevorrichtung zur Viskositätsbestimmung ist durch experimentelles Erzeugen verschiedener Strömungsprofile nachweisbar. Bei einem Ausführungsbeispiel mit einem Ultraschallelement 210 ist das Ultraschallelement 210 optisch nachweisbar.

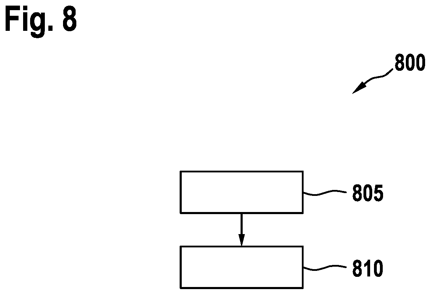

Fig. 8 zeigt ein Ablaufdiagramm eines Verfahrens 800 zum Analysieren einer Viskosität eines Fluids gemäß einem Ausführungsbeispiel. Dabei kann es sich um ein Verfahren 800 handeln, das von einer der anhand der vorangegangenen Figuren beschriebenen Analysevorrichtungen ausführbar ist.

Das Verfahren 800 umfasst einen Schritt 805 des Erkennens und einen Schritt 810 des Bereitstellens. Im Schritt 805 des Erkennens wird die Viskosität des Fluids unter Verwendung zumindest eines Doppler-Parameters eines Doppler-Spektrums des Fluids erkannt. Im Schritt 810 des Bereitstellens wird ein Viskositätssignal bereitgestellt oder gesendet, das die im Schritt 805 des Erkennens erkannte Viskosität repräsentiert.

Die hier vorgestellten Verfahrensschritte 805, 810 können wiederholt sowie in einer anderen als in der beschriebenen Reihenfolge ausgeführt werden.

**Patentansprüche**

1. Herzunterstützungssystem (300) mit einer Aufnahmeschnittstelle (215), mit Einströmöffnungen und mit einer Auslassschnittstelle (220) mit Austrittsöffnungen, mit einer Strömungsmaschine, die einen Impeller aufweist, der mit einem Elektromotor gekoppelt ist, mit einem Anschlusskabel (370) und mit einer an das Anschlusskabel angeschlossenen Kanüle (200), die entlang einer Achse erstreckt und mittig in Aortenklappen (330) anordenbar ist, durch die ein Blutstrom (335) mittels der Strömungsmaschine von der Aufnahmeschnittstelle (215) zu der Auslassschnittstelle (220) gefördert werden kann, **gekennzeichnet durch** eine an ein distales Ende der Kanüle angeschlossenen Spitze (360) und eine Analysevorrichtung (100) zum Analysieren einer Viskosität (105) des Bluts in dem Blutstrom (335), wobei die Analysevorrichtung (100) die folgenden Merkmale aufweist:

   - ein in der Spitze (360) angeordnetes Ultraschallelement (210), das dazu ausgebildet ist, in dem Blut des Blutstroms (335) eine Schallwelle zu erzeugen, die durch einen Zulaufbereich der Aufnahmeschnittstelle (215) hindurch in die Kanüle entlang deren Achse hineinstrahlt, um in Reflexen von Schallwellen aus dem Blut den
   Doppler-Parameter zu erkennen,
   - eine Erkenneinrichtung (110), die dazu ausgebildet ist, unter Verwendung zumindest eines Doppler-Parameters eines Doppler-Spektrums (700) des Bluts in dem Blutstrom (335) die Viskosität (105) des Bluts in dem Blutstrom (335) zu erkennen; und
   - eine Bereitstellungseinrichtung (115), die dazu ausgebildet ist, ein Viskositätssignal (130) bereitzustellen oder zu senden, das die von der Erkenneinrichtung (110) erkannte Viskosität (105) des Bluts in dem Blutstrom (335) repräsentiert.

2. Herzunterstützungssystem (300) nach Anspruch 1, **dadurch gekennzeichnet, dass** die Erkenneinrichtung (110) dazu ausgebildet ist, die Viskosität (105) unter Verwendung eines Funktionszusammenhangs zwischen dem Doppler-Parameter zu der Viskosität (105) zu erkennen.

3. Herzunterstützungssystem nach Anspruch 1 oder 2, **dadurch gekennzeichnet, dass** die Erkenneinrichtung (110) dazu ausgebildet ist, die Viskosität (105) unter Verwendung einer Nachschlagetabelle zu erkennen.

4. Herzunterstützungssystem nach Anspruch 3, **dadurch gekennzeichnet, dass** in der Nachschlagetabelle ein Zusammenhang zwischen dem Doppler-Parameter zu der Viskosität (105) abgelegt ist.

5. Herzunterstützungssystem nach Anspruch 3 oder Anspruch 4, **dadurch gekennzeichnet, dass** die Erkenneinrichtung (110) dazu ausgebildet ist, die Viskosität (105) unter Verwendung einer Interpolation der in der Nachschlagetabelle abgelegten ersten Viskosität und einer in der Nachschlagetabelle abgelegten zweiten Viskosität zu erkennen.

6. Herzunterstützungssystem nach einem der vorangegangenen Ansprüche, **dadurch gekennzeich-**

**net, dass** der Doppler-Parameter einen Doppler-Parameter in der Kanüle (200) repräsentiert.

7. Herzunterstützungssystem nach Anspruch 6, **dadurch gekennzeichnet, dass** die Erkenneinrichtung (110) dazu ausgebildet ist, die Viskosität (105) unter Verwendung zumindest eines Kanülenparameters (r) der Kanüle (200) zu erkennen.

8. Herzunterstützungssystem nach Anspruch 6 oder 7, **dadurch gekennzeichnet, dass** die Strömungseinrichtung (205) im Bereich der Auslassschnittstelle (220) angeordnet ist.

9. Herzunterstützungssystem nach Anspruch 6 oder 7, **dadurch gekennzeichnet, dass** die Strömungseinrichtung (205) an der Auslassschnittstelle (220) angeordnet ist.

10. Herzunterstützungssystem nach einem der Ansprüche 6 bis 9, **dadurch gekennzeichnet, dass** die Erkenneinrichtung (110) dazu ausgebildet ist, die Viskosität (105) unter Verwendung zumindest eines Strömungsparameters eines Strömungsprofils (225) zu erkennen.

11. Herzunterstützungssystem nach Anspruch 10, **dadurch gekennzeichnet, dass** die Erkenneinrichtung (110) dazu ausgebildet ist, eine Strömungsgeschwindigkeit (v) des Bluts in dem Blutstrom (335) durch die Kanüle (200) zu erkennen.

12. Herzunterstützungssystem nach einem der vorangegangenen Ansprüche, **dadurch gekennzeichnet, dass** die Erkenneinrichtung (110) dazu ausgebildet ist, die Viskosität (105) unter Verwendung des Doppler-Parameters zu erkennen, der eine Doppler-Frequenz und/oder eine Breite des Doppler-Spektrums (700) repräsentiert.

13. Verfahren (800) zum Analysieren der Viskosität (105) des Bluts in einem Blutstrom (335), der mittels der Strömungseinrichtung durch die Kanüle in einem nach einem der Ansprüche 1 bis 12 ausgebildeten Herzunterstützungssystem (300) gefördert wird, **gekennzeichnet durch** das Erzeugen einer Schallwelle in dem Blut des Blutstroms mittels des Ultraschallelements (210), die durch einen Zulaufbereich der Aufnahmeschnittstelle (215) hindurch in die Kanüle (200) entlang deren Achse hineinstrahlt, und das Angeben der Viskosität (105) des Bluts in dem Blutstrom (335) unter Verwendung zumindest eines Doppler-Parameters eines Doppler-Spektrums (700) des Blutstroms (335).

**Claims**

1. Cardiac support system (300) comprising a receiving interface (215) having inflow openings and an outlet interface (220) having outlet openings, a turbomachine which has an impeller which is coupled to an electric motor, a connection cable (370), and a cannula (200) which is connected to the connecting cable and which extends along an axis and can be arranged centrally in aortic valves (330), through which a blood flow (335) can be conveyed from the receiving interface (215) to the outlet interface (220) by means of the turbomachine, **characterized by** a tip (360) connected to a distal end of the cannula and an analysis apparatus (100) for analyzing a viscosity (105) of the blood in the blood flow (335), the analysis apparatus (100) having the following features:

   - an ultrasonic element (210) which is arranged in the tip (360) and designed to generate a sound wave in the blood of the blood flow (335), which sound wave radiates through an inlet region of the receiving interface (215) into the cannula along the axis thereof, in order to detect the Doppler parameter in reflections of sound waves from the blood,
   - a detection device (110) which is designed to detect the viscosity (105) of the blood in the blood flow (335) using at least one Doppler parameter of a Doppler spectrum (700) of the blood in the blood flow (335); and
   - a supply device (115) which is designed to supply or send a viscosity signal (130) which represents the viscosity (105) of the blood in the blood flow (335) that is detected by the detection device (110).

2. Cardiac support system (300) according to claim 1, **characterized in that** the detection device (110) is designed to detect the viscosity (105) using a functional relationship between the Doppler parameter and the viscosity (105).

3. Cardiac support system according to either claim 1 or claim 2, **characterized in that** the detection device (110) is designed to detect the viscosity (105) using a lookup table.

4. Cardiac support system according to claim 3, **characterized in that** a relationship between the Doppler parameter and the viscosity (105) is stored in the lookup table.

5. Cardiac support system according to either claim 3 or claim 4, **characterized in that** the detection device (110) is designed to detect the viscosity (105) using an interpolation of the first viscosity stored in the lookup table and a second viscosity stored in the lookup table.

6. Cardiac support system according to any of the preceding claims, **characterized in that** the Doppler parameter represents a Doppler parameter in the cannula (200).

7. Cardiac support system according to claim 6, **characterized in that** the detection device (110) is designed to detect the viscosity (105) using at least one cannula parameter (r) of the cannula (200).

8. Cardiac support system according to either claim 6 or claim 7, **characterized in that** the flow device (205) is arranged in the region of the outlet interface (220).

9. Cardiac support system according to either claim 6 or claim 7, **characterized in that** the flow device (205) is arranged at the outlet interface (220).

10. Cardiac support system according to any of claims 6 to 9, **characterized in that** the detection device (110) is designed to detect the viscosity (105) using at least one flow parameter of a flow profile (225).

11. Cardiac support system according to claim 10, **characterized in that** the detection device (110) is designed to detect a flow velocity (v) of the blood in the blood flow (335) through the cannula (200).

12. Cardiac support system according to any of the preceding claims, **characterized in that** the detection device (110) is designed to detect the viscosity (105) using the Doppler parameter, which represents a Doppler frequency and/or a width of the Doppler spectrum (700).

13. Method (800) for analyzing the viscosity (105) of the blood in a blood flow (335) which is conveyed by means of the flow device through the cannula in a cardiac support system (300) designed according to any of claims 1 to 12, **characterized by** generating a sound wave in the blood of the blood flow by means of the ultrasonic element (210), which sound wave radiates through an inlet region of the receiving interface (215) into the cannula (200) along the axis thereof, and indicating the viscosity (105) of the blood in the blood flow (335) using at least one Doppler parameter of a Doppler spectrum (700) of the blood stream (335).

**Revendications**

1. Système d'assistance cardiaque (300) comportant une interface d'entrée (215) présentant des ouvertures d'admission et une interface d'évacuation (220) présentant des ouvertures de sortie, comportant une machine d'écoulement qui présente une roue accouplée à un moteur électrique, comportant un câble de raccordement (370) et une canule (200) reliée au câble de raccordement, laquelle canule s'étend le long d'un axe et peut être disposée au centre de la valve aortique (330), canule à travers laquelle un flux sanguin (335) peut être acheminé de l'interface d'entrée (215) à l'interface d'évacuation (220) au moyen de la machine d'écoulement, **caractérisé par**
un embout (360) relié à une extrémité distale de la canule et un dispositif d'analyse (100) pour analyser la viscosité (105) du sang dans le flux sanguin (335), le dispositif d'analyse (100) présentant les caractéristiques suivantes :

   - un élément à ultrasons (210) disposé dans l'embout (360) et conçu pour générer une onde sonore dans le sang du flux sanguin (335), laquelle onde sonore rayonne dans la canule le long de son axe, à travers une zone d'admission de l'interface d'entrée (215), de façon à détecter le paramètre Doppler dans les réflexes des ondes sonores provenant du sang,
   - un moyen de détection (110) qui est conçu pour détecter la viscosité (105) du sang dans le flux sanguin (335) à l'aide d'au moins un paramètre Doppler d'un spectre Doppler (700) du sang dans le flux sanguin (335) ; et
   - un moyen de fourniture (115) qui est conçu pour fournir ou envoyer un signal de viscosité (130) qui représente la viscosité (105) du sang dans le flux sanguin (335), laquelle viscosité est détectée par le moyen de détection (110).

2. Système d'assistance cardiaque (300) selon la revendication 1, **caractérisé en ce que** le moyen de détection (110) est conçu pour détecter la viscosité (105) à l'aide d'une relation fonctionnelle entre le paramètre Doppler et la viscosité (105).

3. Système d'assistance cardiaque selon la revendication 1 ou 2, **caractérisé en ce que** le moyen de détection (110) est conçu pour détecter la viscosité (105) à l'aide d'une table à consulter.

4. Système d'assistance cardiaque selon la revendication 3, **caractérisé en ce qu'**une relation entre le paramètre Doppler et la viscosité (105) est mémorisée dans la table à consulter.

5. Système d'assistance cardiaque selon la revendication 3 ou la revendication 4, **caractérisé en ce que** le moyen de détection (110) est conçu pour détecter la viscosité (105) à l'aide d'une interpolation de la première viscosité mémorisée dans la table à consulter et d'une seconde viscosité mémorisée dans la table à consulter.

6. Système d'assistance cardiaque selon l'une des revendications précédentes, **caractérisé en ce que** le paramètre Doppler représente un paramètre Doppler dans la canule (200).

7. Système d'assistance cardiaque selon la revendication 6, **caractérisé en ce que** le moyen de détection (110) est conçu pour détecter la viscosité (105) à l'aide d'au moins un paramètre de canule (r) de la canule (200).

8. Système d'assistance cardiaque selon la revendication 6 ou 7, **caractérisé en ce que** le moyen d'écoulement (205) est disposé dans la zone de l'interface d'évacuation (220).

9. Système d'assistance cardiaque selon la revendication 6 ou 7, **caractérisé en ce que** le moyen d'écoulement (205) est disposé au niveau de l'interface d'évacuation (220).

10. Système d'assistance cardiaque selon l'une des revendications 6 à 9, **caractérisé en ce que** le moyen de détection (110) est conçu pour détecter la viscosité (105) à l'aide d'au moins un paramètre d'écoulement d'un profil d'écoulement (225).

11. Système d'assistance cardiaque selon la revendication 10, **caractérisé en ce que** le moyen de détection (110) est conçu pour détecter une vitesse d'écoulement (v) du sang dans le flux sanguin (335) à travers la canule (200).

12. Système d'assistance cardiaque selon l'une des revendications précédentes, **caractérisé en ce que** le moyen de détection (110) est conçu pour détecter la viscosité (105) à l'aide du paramètre Doppler, lequel paramètre Doppler représente une fréquence Doppler et/ou une largeur du spectre Doppler (700).

13. Procédé (800) d'analyse de la viscosité (105) du sang dans un flux sanguin (335), lequel sang est transporté par le moyen d'écoulement à travers la canule dans un système d'assistance cardiaque (300) conçu selon l'une des revendications 1 à 12, **caractérisé par** la génération d'une onde sonore dans le sang du flux sanguin, au moyen de l'élément à ultrasons (210), laquelle onde sonore rayonne dans la canule (200) le long de son axe, à travers une zone d'admission de l'interface d'entrée (215), et l'indication de la viscosité (105) du sang dans le flux sanguin (335) à l'aide d'au moins un paramètre Doppler d'un spectre Doppler (700) du flux sanguin (335).

# Fig. 1

Fig. 2

EP 3 803 330 B1

# Fig. 3

305

300

355

370

320

365

345

220

100

200

325

330

335

215

210

315

310

360

# Fig. 4

225

$$\frac{dv}{dy}$$

# Fig. 5

210    510    505    500

[A]

# Fig. 6

# Fig. 7

# Fig. 8

800

805

810

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004065143 A1
- DE 102006001180 A1
- US 20120084024 A1
- US 20050126268 A1
- EP 2175770 B1
- US 7591777 B2

**In der Beschreibung aufgeführte Nicht-Patentliteratur**

- **VOLKAN OSEL et al.** Oline Viscosity Measurement of Complex Solutions Using Ultrasound Doppler Velocimetry. *Turk. J. Chem.,* 2006, vol. 30, 297